**Europäisches Patentamt**

**European Patent Office**

**Office européen des brevets**

(19)

(11) Veröffentlichungsnummer : **0 470 117 B1**

(12) # EUROPÄISCHE PATENTSCHRIFT

(45) Veröffentlichungstag der Patentschrift :
26.01.94 Patentblatt 94/04

(51) Int. Cl.$^5$ : **A61K 37/02, C07K 7/10**

(21) Anmeldenummer : **90906216.8**

(22) Anmeldetag : **21.04.90**

(86) Internationale Anmeldenummer :
**PCT/EP90/00644**

(87) Internationale Veröffentlichungsnummer :
**WO 90/12586 01.11.90 Gazette 90/25**

(54) **ARZNEIMITTEL (CALCITONINGEN VERWANDTE PEPTIDE) ZUR BEHANDLUNG EREKTILER DYSFUNKTIONEN.**

(30) Priorität : **27.04.89 DE 3913954**

(43) Veröffentlichungstag der Anmeldung :
**12.02.92 Patentblatt 92/07**

(45) Bekanntmachung des Hinweises auf die
Patenterteilung :
**26.01.94 Patentblatt 94/04**

(84) Benannte Vertragsstaaten :
**AT BE CH DE DK ES FR GB IT LI LU NL SE**

(56) Entgegenhaltungen :
**WO-A-85/01658**
**J. of Anatomy, vol. 149, 1986, (Cambridge, GB)**
**T.L. Lamano Carvalho et al., pp. 121-141**

(56) Entgegenhaltungen :
**The J. of Urology, vol. 141, March 1989, Williams & Wilkins Co. (US) J.A. Owen et al., pp. 546-548**
**Chemical Abstracts, vol. 102, 1985, (Columbus, Ohio, US), p. 165, abstract 198971h**
**Angio, vol. 11, Nr. 5, 1989 (Gräfelfing, DE) J.U. Schwarzer et al., pp. 205-213**

(73) Patentinhaber : **Stief, Georg**
**Rehmenbreiten 6**
**D-30966 Hemmingen (DE)**

(72) Erfinder : **Stief, Georg**
**Rehmenbreiten 6**
**D-30966 Hemmingen (DE)**

(74) Vertreter : **Miller, Andreas**
**Rechtsanwälte Dr. Miller und Partner,**
**Kaiser-Joseph-Strasse 260**
**D-79098 Freiburg (DE)**

**Beschreibung**

Die Erfindung betrifft die Verwendung von "Calcitonin gene related peptides" oder deren Analoge zur Behandlung erektiler Dysfunktionen. Diese Peptide sind bekannt und z.B. beschrieben in EP-A-0 212 432, US 4,530,838 oder US 4,687,839 jedoch mit Wirkungen auf Gedächtnis, Schmerzempfindung oder Senkung des Blutdrucks oder der Magensaftsekretion.

Etwa 5% der Männer im 40. Lebensjahr und 20% im 60. Lebensjahr leiden an einer erektilen Dysfunktion. Durch den Verlust der Potenz wird das körperliche, seelische und soziale Selbstverständnis des Mannes, insbesondere des jungen Mannes, erschüttert. Patienten mit chronischer erektiler Dysfunktion sind in Ihrer Sexualität und Persönlichkeit verunsichert und als krank anzusehen.

Zur Behandlung von bis in die siebziger Jahre überwiegend auf psychische Ursachen zurückgeführten Potenzstörungen wurden neben psychotherapeutischen Maßnahmen Testosterone oder Aphrodisiaka von umstrittenem Wert eingesetzt. Erst durch die Erforschung der Physiologie des Erektionsablaufs wurde festgestellt, daß bei mehr als 60% der Patienten organische Ursachen die Erektionsstörung bewirken, wobei autonome Efferenzen aus dem parasympathischen Anteil des Sakralmarkes, Neurotransmitter, Dilatation der Penisarterien, Relaxation der cavernösen Räume und Konstriktion der Venen eine Rolle spielen. In über 70% der Fälle sind vaskuläre Faktoren ursächlich beteiligt, wie pathologische arterielle Blutversorgungen oder abnorm gesteigerter venöser Abfluß aus den kavernosen Räumen. Neurogene Störungen sind zu etwa 20% beteiligt.

Die orale Therapie dieser organisch bedingten Dysfunktionen mit vasoaktiven Substanzen wie Yohimbin, Phenoxybenzamin, Terbutalin, Bethanechol, Levodopa, Verapamil oder Theophyllin erwies sich als erfolglos. Neben der Anwendung von Prothesenimplantationen oder Revaskularisierungsoperationen erwies sich eine intrakavernöse Injektion von Papaverin (Virag, Lancet, 2, 938, 1982), dem $\alpha$-Rezeptorenblocker Phenoxybenzamin (Brindley, Br. J. Psychiatr., 143, 332, 1983) und eine Kombination von Papaverin und dem $\alpha$-Rezeptorenblocker Phentolamin (Stief, Urologe A, 25, 63, 1986) als erfolgreich. Letztere Therapiemethode läßt sich vom Patienten selbständig durchführen und wird auch als Schwellkörper-Autoinjektionstherapie (SKAT) bezeichnet.

Nachteilig erwiesen sich jedoch eine teilweise unerwünscht verlängerte Erektion mit der Gefahr des Priapismus bei der Verwendung von Papaverin, unerwünschte Schmerzhaftigkeit bei der Anwendung von Phenoxybenzamin sowie eine mögliche Cancerogenität dieser Verbindung.

Im Tierexperiment (Cynomolgus) wurden zudem bei 1-2 Intracavernös-Injektionen Papaverin wöchentlich über 12 Monate ausgedehnte Fibrosierungen weiter Teile des Schwellkörpers festgestellt, was beim Menschen zu äußerst negativen Langzeitergebnissen führen würde, da bei einer Fibroisierung des corpus cavernosum keine Erektion mehr erfolgen kann.

Die Anwendung von Acetylcholin ist bei nur kurzzeitiger Erektion mit starken systemischen Nebenwirkungen verbunden und die Injektion von Prostaglandin $E_1$ wird von den Patienten wegen zu großer Schmerzhaftigkeit abgelehnt.

Aufgabe der Erfindung war daher die Entwicklung und Herstellung von Arzneimitteln zur Behandlung von neurogenen, arteriellen, neurotransmitterbedingten, myopathischen, venösen oder psychogenen erektilen Dysfunktionen bei Säugetieren, bevorzugt beim Menschen, ohne die genannten Nebenwirkungen.

Überraschenderweise wurde nun gefunden, daß die intracavernöse Injektion eines körpereigenen Peptamids gemäß der Formel I,

```
H-Ala-Cys-Asp-Thr-Ala-Thr-Cys-Val-Thr-His-Arg-

Leu-Ala-Gly-Leu-Leu-Ser-Arg-Ser-Gly-Gly-Val-Val-      (I)

Lys-Asn-Asn-Phe-Val-Pro-Thr-Asn-Val-Gly-Ser-Lys-


Ala-Phe-NH2
```

dessen Struktur durch alternatives Spleißen vom Calcitonin-Gen kodiert wird, das heißt ein sogenanntes "calcitonin gene related peptide" (CGRP), welches starke vasodilatatorische Eigenschaften aufweist, zu einer Erektion mit vollständiger Rigidität führt. Da zudem das humane "calcitonin gene related peptid" (h-CGRP) ubiquitär im Organismus vorkommt ist die therapeutische Anwendung ohne die Gefahr einer nachfolgenden Fibroisierung möglich.

Gegenstand der Erfindung ist daher die Verwendung von "calcitonin gene related peptides", nachfolgend als CGRP bezeichnet, deren Analoge oder diese als Teilsequenz eines größeren Peptids oder als Gesamtsequenz, bevorzugt die Aminosäuresequenz der allgemeinen Formel II

$$R^1-CH-CH_2 \underline{\hspace{1cm}} X \underline{\hspace{1cm}} Y \underline{\hspace{1cm}} CH_2$$
$$| \hspace{5cm} |$$
$$CO-Q-Thr-Ala-Thr-NHCH-CO-Val-Thr-His-Arg-Leu-Ala-$$
$$A-B-Leu-Ser-Arg-Ser-Gly-Gly-D-E-Lys-G-Asn-Phe-Val- \hspace{1cm} (II)$$
$$Pro-Thr-Asn-Val-Gly-Ser-K-L-M-R^3$$

ist, in welcher $R^1$ entweder Wasserstoff oder ein Rest der allgemeinen Formel III,

$$R^2-T- \hspace{1cm} (III)$$

ist, worin T Ala oder Ser und $R^2$ Wasserstoff oder eine Acylgruppe mit 1 bis 4 C-Atomen, bevorzugt eine Acetylgruppe ist, und X und Y unabhängig voneinander eine Methylengruppe oder Schwefel darstellen und Q entweder Asp oder Asn ist,

| | |
|---|---|
| A | ist Asp, Asn, Glu oder Gly |
| B | ist Phe oder Leu |
| D | ist Met oder Val |
| E | ist Gly oder Val |
| G | ist Asn, Ser oder Asp |
| K | ist Lys oder Glu und |
| L und M | jede beliebige Aminosäure sein kann, bevorzugt |
| L | jedoch Ala, Phe, Pro, Glu, Ser, Ile, Leu, Val, Tyr, Hypro, Gln, Hse, Thr, Asp oder Asn, besonders bevorzugt Ala, Val Leu, Ile, Thr, Asp, Asn, Glu oder Gln und |
| M | bevorzugt Phe, Pro, Hypro, Tyr, Ala, Val, Leu, Ile, Ser, Thr, Asp, Asn, Glu oder Gln ist, und |
| $R^3$ | eine Hydroxy- oder Aminogruppe oder eine weitere beliebige Aminosäure, bevorzugt Gly oder Tyr, oder eine der Peptidsequenzen |

$$-Gly-Arg-Arg-Arg-Arg-Asp-Leu-Gln-Ala,$$

$$-Gly-Arg-Arg-Arg-Arg \text{ oder}$$

$$-Gly-Lys-Lys-Arg \text{ bedeutet,}$$

sowie deren homologe sowie Teilsequenzen dieser Peptide, die um bis zu 10 Aminosäuren am C-terminalen Ende der Kette verkürzt sein können und die pharmakologisch unbedenklichen Salze dieser Peptide zur Herstellung von Arzneimitteln zur Behandlung erektiler Dysfunktionen bei Säugetieren, bevorzugt beim Menschen, Arzneimittel, die die genannten Peptide enthalten, sowie die Behandlung erektiler Dysfunktionen bei Säugetieren und Menschen mittels der o.g. Peptide.

Bevorzugt ist das Peptamid der Formel I, das humane CGRP.

Für den Fall, daß in einem Peptid der allgemeinen Formel II X und Y gleichzeitig Schwefel bedeuten, können neben den bevorzugten intramolekularen Disulfidbrücken auch Peptide durch Ausbildung von intermolekularen Disulfidbrücken als Dimere vorliegen, wobei eine Kopf-Kopf, das heißt parallele, bevorzugt jedoch eine Kopf-Schwanz, das heißt antiparallele Verknüpfung möglich ist.

Den internationalen Nomenklaturregeln entsprechend bezeichen die Abkürzungen für die oben genannten Aminosäuren die freie Säure und die L- oder D-Konfiguration, bevorzugt jedoch die L-Konfiguration, wobei die $\alpha$-Aminogruppe an der linken und die Carboxygruppe an der rechten Seite zu denken ist. Das Fehlen eines Wasserstoff-Atoms an der $\alpha$-Aminogruppe bezeichnet ein Bindestrich an der linken Seite der Abkürzung, das Fehlen der Hydroxygruppe in der Carboxygruppe ein Bindestrich an der rechten Seite.

Die Erfindung betrifft auch die Verwendung der aus galenischen Gründen in die pharmakologisch verträgliche Salze übergeführten Verbindungen der allgemeinen Formel II. Die Salze werden in üblicher Weise durch Neutralisation der Basen mit anorganischen oder organischen Säuren erhalten.

Als anorganische Säuren kommen zum Beispiel Salzsäure, Schwefelsäure, Phosphorsäure oder Bromwasserstoffsäure, als organische Säuren zum Beispiel Carbon-, Sulfo- oder Sulfonsäuren wie Essigsäure, Weinsäure, Milchsäure, Propionsäure, Glykolsäure, Malonsäure, Maleinsäure, Fumarsäure, Gerbsäure, Succinsäure, Alginsäure, Benzoesäure, 2-Phenoxybenzoesäure, 2-Acetoxybenzoesäure, Zimtsäure, Mandelsäure, Zitronensäure, Äpfelsäure, Salicylsäure, 3-Aminosalicylsäure, Ascorbinsäure, Embonsäure, Nicotinsäure, Isonicotinsäure, Oxalsäure, Aminosäuren, Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure in Frage.

Auch können die Peptide, die mindestens eine Carboxyl- und mindestens eine basische Gruppe, zum Beispiel ein Aminogruppe enthalten, in der Form ihrer inneren Salze Verwendung finden.

Ebenso können diejenigen der oben genannten Peptide verwendet werden, die aufgrund einer freien Carboxylgruppe in Metall- oder Ammoniumsalze übergeführt worden sind. Als Metallsalze kommen zum Beispiel die Zink, Eisen, Natrium, Kalium, Barium, Aluminium, Magnesium oder Calciumsalze, als Ammoniumsalze die Salze mit Ammoniak oder organischen Aminen in Frage, wobei aliphatische, cycloaliphatische, cycloaliphatisch-aliphatische oder araliphatische primäre, sekundäre oder tertiäre Mono-, Di- oder Polyamine sowie hetercyclische Basen verwendet werden können, wie Alkylamine mit jeweils 1 bis 6 C-Atomen in den Alkylgruppen, zum Beispiel Triethylamin, Hydroxyalkylamine mit jeweils 1 bis 6 C-Atomen in den Alkylgruppen wie zum Beispiel 2-Hydroxyethylamin, Bis-(2-hydroxyethyl)-amin, 2-Hydroxyethyldiethyl-amin oder Tri-(2-hydroxyethyl)-amin, oder auch basische aliphatische Ester von Carbonsäuren wie zum Beispiel 4-Aminobenzoesäure-2-diethylaminoethylester, Alkylenamine wie zum Beispiel 1-Ethylpiperidin, Cykloalkylamine wie beispielsweise Dicyclohexylamin, oder Benzylamine wie N,N-Dibenzylethylendiamin, oder auch Basen vom Pyridintyp wie Pyridin, Collidin oder Chinolin.

Die erfindungsgemäßen Arzneimittel enthalten neben den üblichen Hilfs-, Träger- und Zusatzstoffen eine wirksamen Dosis von Verbindungen der allgemeinen Formel II oder deren Salze zur Behandlung der genannten Dysfunktionen. Die Dosierung ist abhängig von Spezies, Körpergewicht, Alter, individuellem Zustand und Applikationsart.

Als Applikationsformen kommen sowohl parenterale als auch topische Zubereitungen wie beispielsweise Lotionen, Cremes, Lösungen, Gele, Sprays, elastische Flüssig-Pflaster, transdermale Systeme oder Beschichtungen für Kondome in Frage.

Zubereitungen zur parenteralen Applikation enthalten 0,5 µg bis 1 mg, bevorzugt 5 bis 500 µg der Verbindungen der allgemeinen Formel II pro Dosiseinheit und können in separaten Dosiseinheitsformen wie z. B. Ampullen oder Vials vorliegen. Vorzugsweise werden Lösungen des Wirkstoffes verwendet, bevorzugt wässrige Lösungen und vor allem isotonische Lösungen, aber auch Suspensionen. Diese Injektionsformen können als Fertigpräparat zur Verfügung gestellt werden oder erst direkt vor der Anwendung durch Mischen der wirksamen Verbindung, zum Beispiel des Lyophilisats, gegebenenfalls mit weiteren festen Trägerstoffen, mit dem gewünschten Lösungs- oder Suspensionsmittel zubereitet werden.

Parenterale wie topische Formen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze zur Regelung des osmotischen Drucks oder zur Pufferung und/oder Viskositätsregulatoren enthalten.

Derartige Zusätze sind zum Beispiel Tartrat- und Citrat-Puffer, Ethanol, Komplexbildner (wie Äthylendiamintetraessigsäure und deren nicht-toxische Salze). Zur Regelung der Viskosität kommen hochmolekulare Polymere in Frage wie beispielweise flüssiges Polyethylenoxid, Carboxymethylcellulosen, Polyvinylpyrrolidone, Dextrane oder Gelatine. Feste Trägerstoffe sind zum Beispiel Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol.

Ölige Suspensionen für parenterale oder topische Anwendungen können vegetabile synthetische oder semisynthetische Öle wie beispielsweise flüssige Fettsäureester mit jeweils 8 bis 22 C-Atomen in den Fettsäureketten, zum Beispiel Palmitin-, Laurin-, Tridecyl-, Margarin-, Stearin-, Arachin-, Myristin-, Behen-, Pentadecyl-, Linol-, Elaidin-, Brasidin-, Eruca- oder Ölsäure, die mit ein- bis dreiwertigen Alkoholen mit 1 bis 6 C-Atomen wie beispielsweise Methanol, Ethanol, Propanol, Butanol, Pentanol oder deren Isomere, Glycol oder Glycerol verestert sein. Derartige Fettsäureester sind beispielsweise handelsübliche Miglyole, Isopropylmyristat, Isopropylpalmitat, Isopropylstearat, PEG 6-Caprinsäure, Capryl/Caprinsäureester von gesättigten Fettalkoholen, Polyoxyethylenglyceroltrioleate, Ethyloleat, wachsartige Fettsäureester wie künstliches Enten-

EP 0 470 117 B1

bürzeldrüsenfett, Kokosfettsäure-isopropylester, Ölsäureoleylester, Ölsäuredecylester, Milchsäureethylester, Dibuthylphthalat, Adipinsäurediisopropylester, Polyol-Fettsäureester u. a. Ebenso geeignet sind Silikonöle verschiedener Viskosität oder Fettalkohole wie Isotridexylalkohol, 2-Octyldodecanol, Cetylstearyl-Alkohol oder Oleylalkohol, Fettsäuren wie beispielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden. Die genannten Stoffe haben zudem die Eigenschaften eines Spreitmittels, das heißt es erfolgt eine besonders gute Verteilung auf der Haut.

Als Lösungsmittel, Gelbildner und Lösungsvermittler kommen in Frage Wasser oder mit Wasser mischbare Lösungsmittel. Geeignet sind zum Beispiel Alkohole wie beispielsweise Ethanol oder Isopropylalkohol, Benzylalkohol, 2-Octyldodecanol, Polyethylenglykole, Phthalate, Adipate, Propylenglyklol, Glycerin, Di- oder Tripropylenglykol, Wachse, Methylcellosolve, Cellosolve, Ester, Morpholine, Dioxan, Dimethylsulfoxid, Dimethylformamid, Tetrahydrofuran, Cyclohexanon etc.

Als Filmbildner können Celluloseether verwendet werden, die sich sowohl in Wasser als auch in organischen Lösungsmitteln lösen bzw. anquellen können und nach dem Trocknen eine Art Film bilden, wie beispielsweise Hydroxypropylcellulose, Methylcellulose, Ethylcellulose oder lösliche Stärken.

Mischformen zwischen Gel- und Filmbildnern sind durchaus ebenfalls möglich. Hier kommen vor allem ionische Makromoleküle zur Anwendung, wie beispielsweise Natriumcarboxymethylcellulose, Polyacrylsäure, Polymethacrylsäure und deren Salze, Natriumamylopektinsemiglykolat, Alginsäure oder PropylenglykolAlginat als Natriumsalz, Gummi arabicum, Xanthan-Gummi, Guar-Gummi oder Carrageenan.

Als weitere Formulierungshilfsmittel können eingesetzt werden: Glycerin, Paraffin unterschiedlicher Viskosität, Triethanolamin, Collagen, Allantoin, Novantisolsäure, Parfümöle.

Laurylsulfat, Fettalkoholethersulfaten, Di-Na-N-lauryl-$\beta$-iminodipropionat, polyoxyethyliertes Rizinusöl oder Sorbitan-Monooleat, Sorbitan-Monostearat, Cetylalkohol, Lecithin, Glycerinmonostearat, Polyoxyethylenstearat, Alkylphenolpolyglykolether, Cetyltrimethylammoniumchlorid oder Mono-/Dialkylpolyglykoletherorthophosphorsäuremonoethanolaminsalze.

Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Zur Förderung der Penetration enthalten transdermale Formulierungen vorzugsweise organische, gut hautverträgliche Lösungsmittel wie Ethanol, Methylpyrrolidon, Polyethylenglykol, Oleylalkohol, Octanol, Linolsäure, Triacetin, Propylenglykol, Glycerin, Solketal oder Dimethylsulfoxid.

Die Herstellung, Abfüllung und die Verschließung der Präparate erfolgt unter den üblichen antimikrobiellen und aseptischen Bedingungen. Auch für topischen beziehungsweise transdermalen Einsatz erfolgt eine Abpackung möglichst in separaten Dosiseinheiten zur Erleichterung der Handhabung, auch hier wie bei parenteralen Formen gegebenenfalls aus Stabilitätsgründen durch separate Abpackung der Wirkstoffe beziehungweise deren Kombinationen als Lyophilisat, gegebenenfalls mit festen Trägerstoffen, und den erforderlichen Lösungsmitteln etc.

Ein weiterer Aspekt der Erfindung ist die Verwendung der Verbindungen der allgemeinen Formel I in Kombination mit synergistisch wirkenden Substanzen wie Adenosin, Vitaminen, zum Beispiel Vitamin A oder H, Prostaglandinen, zum Beispiel $E_1$, mit Calcium-Antagonisten wie Nifedipin, Verapamil, Diltiazem, Gallopamil, Niludipin, Nimodipin, Nicardipin, Prenylamin, Fendilin, Terodilin, Nisaldipin, Nitrendipin oder Perhexilin.

Weitere Kombinationsmöglichkeiten bestehen mit $\alpha$-Rezeptorenblockern, beispielsweise Phentolaminmethansulfonat, Phenoxybenzamin oder Minoxidil, oder Relaxantien der glatten Muskulatur wie beispielsweise Papaverin.

Die nachfolgenden Ausführungsbeispiele zu möglichen Arzneizubereitungen sind ausschließlich zur Veranschaulichung der erfinderischen Lehre, jedoch keineswegs als deren Limitierung gedacht.

Beispiel 1 - Injektionslösung

50 mg humanes CGRP werden mit 750 mg NaCl in destilliertem Wasser gelöst, mit 1N HCl auf pH 3,7 eingestellt und mit destilliertem Wasser auf 100 ml aufgefüllt und in 0,5 ml-Ampullen abgepackt.

Beispiel 2 - Lösung zur topischen Applikation

Aus 500 mg CGRP, 2 ml Isopropylmyristat und 10 ml Ethanol wird eine Lösung zur topischen Applikation bereitet und zu Dosiseinheiten von jeweils 2 ml abgepackt.

### Beispiel 3 - Transdermales Pflaster

10 g Linolsäure und 90 g Propylenglykol werden gemischt. In dieser Mischung werden 5 g CGRP gelöst. Mit der Lösung werden einseitig mit Kunststoff beschichtete Gazequadrate getränkt und in Aluminiumfolie versiegelt.

### Beispiel 4 - Streichfähiges Gel

94 g gereinigtes Wasser werden auf 70°C erwärmt und mit 10 g CGRP versetzt. Nach Zugabe von 0,2 g p-Hydroxybenzoesäureester werden in die erhaltene Lösung 5 g Methylhydroxyethylcellulose dispergiert. Dann wird unter Rühren abgekühlt. Nach dem Erkalten erhält man ein hochviskoses Gel mit einer Viskosität dispergiert. Dann wird unter Rühren abgekühlt. Nach dem Erkalten erhält man ein hochviskoses Gel mit einer Viskosität von 90 Pa.s.

### Beispiel 5 - Öl-in-Wasser Emulsion

In einem ersten Ansatz werden 7 g einer Mischung bestehend aus gesättigten Fettsäuren, Fettalkoholen, Wollwachs, Mineralölen und nichtionogenen Emulgatoren zusammen mit 2,5 g Polyethylenglykol-Glycerolfettsäureester, 3 g Monoglyceriden der Stearin- und Palmitinsäure, 0,3 g Cetylalkohol und 3,0 g Isopropylpalmitat durch Erwärmen auf 70°C im Wasserbad homogen geschmolzen. In einem zweiten Ansatz werden 80 g gereinigtes Wasser unter Rühren mit 3 g Propylenglykol gemischt und auf 70°C erwärmt. Das so erhaltene Gemisch wird dann mit 5 g CGRP und 200 mg eines Konservierungsmittels versetzt. Die erhaltene klare Lösung wird unter Rühren bei 70°C in den ersten Ansatz einemulgiert. Die so erhaltene Emulsion wird auf 40°C abgekühlt und der durch Verdunstung erlittene Wasserverlust ergänzt. Die auf 30°C agbekühlte Emulsion wird dann abgefüllt.

### Beispiel 6 - Flüssig-Pflaster

5 g CGRP werden in einer Mischung von 5 g Benzylalkohol, 6 g Isopropylstearat oder einer gleiche Menge eines Isopropylmyristat/Isopropylpalmitat/Isopropylstearat-Gemisches 10 g Vinylpyrrolidon/Vinylacetat-Copolymer und 89 g Isopropanol gelöst. Die Lösung kann in separaten Dosiseinheiten zur flüssigen Applikation oder als Spray mit den üblichen Treibmitteln abgepackt werden.

### Beispiel 7 - Öl-Wasser-Emulsion

Nach den üblichen Methoden wird eine Mischung von 5 g CGRP, 9 g eines Gemisches von Mono- und Diglyceriden der Palmitin- und Stearinsäure, 3 g Cetylstearylalkohol mit ca. 12 Mol Ethylenoxid, 10 g 2-Octyldodecanol, 5 g dickflüssigem Paraffin, 5 g Benzylalkohol, 500 mg PHB-Ester und entmineralisiertem Wasser ad 100 g hergestellt.

### Beispiel 8 - Creme weicher Konsistenz

Eine derartige Creme enthält beispielsweise
5 g CGRP, 4 g Mono- und Diglyceride von Palmitin und Stearinsäure, 4 g Cetylpalmitat, 1 g Cetylstearylalkohol mit ca. 12 Mol Ethylenoxid, 1 g Cetylstearylalkohol mit ca. 30 Mol Ethylenoxid, 5 g Isopropylmyristat-/Isopropylpalmitat-/Isopropylstearat-Gemisch, 0,5 g schwach vernetzte Polyacrylsäure von extrem hohem MG, 0,11 g Natriumhydroxid 45%ig, 3 g Glycerin und entmineralisiertes Wasser ad 100 g.

### Beispiel 9 - Nichtfettende Emulsion

Eine Mischung von 2,5 g Ölsäuredecylester, 2,5 g Isopropylmyristat, 4 g dünnflüssigem Paraffin, 0,9 g Polyethylenstearat, 0,6 g Sorbitan- und Glycerinfettsäureester werden 10 min bei 70°C gerührt und geschmolzen. Die geschmolzene Mischung wird in eine 75°C warme Lösung von 50 g entmineralisiertem Wasser, 500 mg CGRP und 100 mg Allantoin unter Rühren gegeben und auf 45°C abgekühlt. Bei dieser Temperatur wird ein Carbopolschleim aus 10 g Ethanol, 0,7 g Carbopol 934 (schwach vernetzte Polyacrylsäure) und 22,95 g entmineralisiertem Wasser gegeben, welche mit Turrax dispergiert wurde, anschließend 2 h gequollen ist und mit 0,15 g 45%iger Natronlauge neutralisiert wurde. Beim Erreichen von 40°C wird dann noch 1 g Collagen zugegeben. Zuletzt wird die Rohemulsion, gegebenenfalls nach einer Zugabe von 0,6 g Parfümöl, bei 20 bis 25°C im Hochdruckhomogenisator homogenisiert.

Beispiel 10 - Gelatine-Lösung

Für eine Gelatine-Lösung werden 10 µg CGRP, 150 mg Gelatine, 4,7 mg Phenol mit destilliertem Wasser zu 1 ml aufgefüllt und in Vials zu 1 ml abgefüllt.

Beispiel 11 - Spray

In einer Mischung von 3,5 ml Miglyol 812 und 0,08 g Benzylalkohol werden 200 µg CGRP suspendiert. Diese Suspension wird in einen Behälter mit Dosierventil eingefüllt. Es werden nun 5 ml Freon 12 unter Druck durch das Ventil in den Behälter gefüllt. Durch Schütteln wird das Freon in der Miglyol-Benzylalkoholmischung gelöst.

Die Wirksamkeit der Arzneimittel für die erfindungsgemäße Lehre wird durch folgende pharmakologischen Untersuchungen belegt:

Die erforderlichen in-vivo-Versuche wurden an sieben Cynomolgus-Affen zwischen 4,3 bis 8,3 kg Körpergewicht unter Ketamin-Anästhesie (30 mg/kg intramuskular) durchgeführt. Die Affen wurden in Rückenlage positioniert. Unter sterilen Bedingungen wurde eine 21-G Butterfly-Kanüle bilateral im distalen Schwellkörper plaziert. Eine Nadel wurde mit einem Statham-Druckwandler (Model P23 BC) für die Aufzeichnung des intracavernalen Druckes verbunden, die andere zur intracavernösen Injektion oder Perfusion. Die penile Tumeneszenz wurde visuell durch zwei Forscher verfolgt und aufgezeichnet. Eine Klassifikation der Tumeneszenz erfolgte gemäß der Parameter: E 0 = keine Tumeneszenz; E 1 = leichte Tumeneszenz; E 2 = teilweise Tumeneszenz; E 3 = volle Tumeneszenz. Eine Durchflußmessung der cavernalen Arterien erfolgte mittels Doppelultraschall bei 4 Affen. Puls und Blutdruck wurden mittels Dopplermessung (Parks Medical Electronics) an der Radialarterie mit Hilfe einer pediatrischen Blutdruckmanschette vorgenommen.

In einer Pilot-Studie wurden 50, 500 und 2.500 ng h-CGRP (Sigma Chemical Co., St. Louis, MO) intracavernös bei zwei Affen injiziert. 50 ng induzierten nur eine leichte, kurzzeitige Tumeneszenz. 2.500 ng h-CGRP senkten den systemischen Blutdruck unter 35 cm $H_2O$. Das erektile Verhalten war dabei jedoch ähnlich der 500 ng Applikation, nur länger andauernd. Die weiteren Versuche wurden deshalb mit Dosierungen von 500 ng durchgeführt und zur Sicherstellung der Reproduzierbarkeit an einem zweiten Tag wiederholt.

Zunächst wurde nach der intracavernösen Injektion ein Anstieg des arteriellen Durchflusses beobachtet, dem eine Tumeneszenz des Penis folgte und etwa eine Minute danach ein Anstieg des intracavernösen Druckes. Vor der Injektion war eine Messung der Durchflußgeschwindigkeit der cavernösen Arterie nicht möglich. Im Mittel wurde eine maximale Durchflußgeschwindigkeit 4 min nach der CGRP-Injektion beobachtet, die nach weiteren 3-4 min wieder absank. 32 bis 69 min (49 min im Mittel) nach der intracavernösen Injektion ließ sich kein arterieller Fluß mehr feststellen.

Ein Tumeneszenzanstieg des Penis wurde 30 bis 60 sec nach der CGRP-Injektion beobachtet und maximale Tumeneszenz und Verlängerung (E 3) 4 min nach der Injektion zur Zeit des maximalen arteriellen Durchflusses bis zu 15 min nach der Injektion. Die Tumeneszenz nahm dann schrittweise ab bis nach einem Mittelwert von 32 min kein Unterschied zwischen der Tumeneszenz vor und nach der Injektion festgestellt wurde. Der intracavernöse Druck vor der CGRP-Injektion betrug 24 bis 45 (im Mittel 34) cm $H_2O$, 90 bis 120 sec nach der Injektion 62 bis 94 (im Mittel 78) cm $H_2O$, und sank nach 4 min innerhalb 1 min auf 40 bis 54 (im Mittel 47) cm $H_2O$. Dieser Druck sank dann innerhalb von 36 min auf den Ursprungswert ab.

**Patentansprüche**

1.    Verwendung von "calcitonin gene related peptides" mit der allgemeinen Formel II

$$R^1-CH\text{--}CH_2\text{------}X\text{------}Y\text{------}CH_2$$
$$|\qquad\qquad\qquad\qquad\qquad\qquad\qquad|$$
$$CO-Q-Thr-Ala-Thr-NHCH-CO-Val-Thr-His-Arg-Leu-Ala-$$
$$A-B-Leu-Ser-Arg-Ser-Gly-Gly-D-E-Lys-G-Asn-Phe-Val-\qquad (II)$$
$$Pro-Thr-Asn-Val-Gly-Ser-K-L-M-R^3$$

in welcher R¹ entweder Wasserstoff oder ein Rest der allgemeinen Formel III,

$$R^2\text{-T-} \qquad \text{(III)}$$

ist, worin T Ala oder Ser und R² Wasserstoff oder eine Acylgruppe mit 1 bis 4 C-Atomen, bevorzugt eine Acetylgruppe ist, und X und Y unabhängig voneinander eine Methylengruppe oder Schwefel darstellen und Q Asp oder Asn ist,

| | |
|---|---|
| A | ist Asp, Asn, Glu oder Gly |
| B | ist Phe oder Leu |
| D | ist Met oder Val |
| E | ist Gly oder Val |
| G | ist Asn, Ser oder Asp |
| K | ist Lys oder Glu und |
| L und M | jede beliebige Aminosäure sein kann, und |
| R³ | eine Hydroxy- oder Aminogruppe oder eine weitere beliebige Aminosäure, oder eine der Peptidsequenzen |

```
-Gly-Arg-Arg-Arg-Arg-Asp-Leu-Gln-Ala,


-Gly-Arg-Arg-Arg-Arg oder


-Gly-Lys-Lys-Arg bedeutet,
```

sowie von Peptiden mit Teilsequenzen der Formel II, bei denen bis zu 10 Aminosäuren des C-terminalen Endes fehlen oder von größeren Peptiden, in denen Peptide der Formel II eine Teilsequenz darstellen, sowie von pharmakologisch unbedenklichen Salzen dieser Peptide zur Herstellung von Arzneimitteln zur Behandlung erektiler Dysfunktionen bei Säugetieren und Menschen.

2. Verwendung von humanem CGRP gemäß Formel I

```
H-Ala-Cys-Asp-Thr-Ala-Thr-Cys-Val-Thr-His-Arg-

Leu-Ala-Gly-Leu-Leu-Ser-Arg-Ser-Gly-Gly-Val-Val-      (I)

Lys-Asn-Asn-Phe-Val-Pro-Thr-Asn-Val-Gly-Ser-Lys-

Ala-Phe-NH₂
```

zur Herstellung von Arzneimitteln zur Behandlung erektiler Dysfunktionen bei Säugetieren und Menschen gemäß Anspruch 1.

3. Verwendung von Peptiden gemäß der Ansprüche 1 oder 2 in Kombination mit einem oder mehreren Wirkstoffen aus den Wirkstoffgruppen Adenosin, Vitaminen, Prostaglandinen, Calcium-Antogonisten, α-Rezeptorenblocker, Relaxantien der glatten Muskulatur zur Herstellung von Arzneimitteln zur Behandlung erektiler Dysfunktionen bei Säugetieren und Menschen.

4. Verwendung von Peptiden und Wirkstoffkombinationen entsprechend der Ansprüche 1 bis 3, dadurch gekennzeichnet, daß 0,5 µg bis 5 mg der Peptide gemäß der Ansprüche 1 bis 3 pro Dosiseinheit zur Behandlung erektiler Dysfunktionen bei Säugetieren und Menschen eingesetzt werden.

## Claims

1. Use of "calcitonin gene-related peptides" of the general formula (II):

$$R^1\text{-CH--CH}_2\text{---X---Y---CH}_2$$

CO-Q-Thr-Ala-Thr-NHCH-CO-Val-Thr-His-Arg-Leu-Ala-

A-B-Leu-Ser-Arg-Ser-Gly-Gly-D-E-Lys-G-Asn-Phe-Val-     (II)

Pro-Thr-Asn-Val-Gly-Ser-K-L-M-R$^3$

wherein $R^1$ is either a hydrogen atom or a radical of the general formula (III):

$$R^2\text{-T-}   \text{(III)}$$

wherein T is Ala or Ser and $R^2$ is a hydrogen atom or an acyl radical containing up to 4 carbon atoms and preferably an acetyl radical and X and Y, independently of one another, are methylene radicals or sulphur atoms and Q is Asp or Asn,

| | |
|---|---|
| A | is Asp, Asn, Glu or Gly, |
| B | is Phe or Leu, |
| D | is Met or Val, |
| E | is Gly or Val, |
| G | is Asn, Ser or Asp, |
| K | is Lys or Glu and |
| L and M | can be any desired amino acid and |
| $R^3$ | is a hydroxyl or amino group or any further desired amino acid or a peptide of the sequence |

-Gly-Arg-Arg-Arg-Arg-Asp-Leu-Gln-Ala,

-Gly-Arg-Arg-Arg-Arg  or

-Gly-Lys-Lys-Arg,

as well as of the analogues or partial sequences thereof in which up to 10 amino acids of the C-terminal end can be omitted or also large peptides in which the peptides of general formula (II) represent a partial sequence, as well as of the pharmacologically acceptable salts of these peptides for the preparation of pharmaceutical compositions for the treatment of erectile dysfunctions in mammals and men.

2.   The use of human CGRP of the formula (I)

H-Ala-Cys-Asp-Thr-Ala-Thr-Cys-Val-Thr-His-Arg-

Leu-Ala-Gly-Leu-Leu-Ser-Arg-Ser-Gly-Gly-Val-Val-     (I)

Lys-Asn-Asn-Phe-Val-Pro-Thr-Asn-Val-Gly-Ser-Lys-

Ala-Phe-NH$_2$

for the preparation of pharmaceutical compositions for the treatment of erectile dysfunctions in mammals and men according to claim 1.

3.   The use of the peptides according to claims 1 or 2 in combination with one or more active materials selected from the group adenosine, vitamins, prostaglandins, calcium antagonists, α-receptor blockers and relaxants of the smooth musculature for the preparation of pharmaceutical compositions for the treatment of erectile dysfunctions in mammals and men.

4. The use of peptides and active material combinations according to claims 1 to 3, wherein there are used 0.5 µg to 5 mg of the peptides according to any of claims 1 to 3 per dosage unit for the treatment of erectile dysfunctions in mammals and men.

**Revendications**

1. Utilisation de peptides en rapport avec le "gène de la calcitonine " de formule générale II

$$R^1-CH--CH_2----X----Y---CH_2$$
$$|\qquad\qquad\qquad\qquad\qquad\qquad |$$
$$CO-Q-Thr-Ala-Thr-NHCH-CO-Val-Thr-His-Arg-Leu-Ala-$$
$$A-B-Leu-Ser-Arg-Ser-Gly-Gly-D-E-Lys-G-Asn-Phe-Val-\qquad (II)$$
$$Pro-Thr-Asn-Val-Gly-Ser-K-L-M-R^3$$

dans laquelle R¹ représente, soit un atome d'hydrogène, soit un radical de formule générale III

$$R^2-T-$$

dans laquelle T représente Ala ou Ser et R² représente un atome d'hydrogène ou un groupe acyle ayant de 1 à 4 atomes de carbone et, de préférence, un groupe acétyle, tandis que X et Y représentent, indépendamment l'un de l'autre, un groupe méthylène ou un atome de soufre, et Q représente Asp ou Asn,

A          représente Asp, Asn ou Gly
B          représente Phe ou Leu
D          représente Met ou Val
E          représente Gly ou Val
G          représente Asn, Ser ou Asp
K          représente Lys ou Glu et
L et M          peuvent être n'importe quel acide aminé,
R³          représente un groupe hydroxy ou amino ou un autre acide aminé quelconque et, de préférence, Gly ou Tyr, ou l'une des séquences de peptides

$$-Gly-Arg-Arg-Arg-Arg-Asp-Leu-Gln-Ala;$$

$$-Gly-Arg-Arg-Arg-Arg \quad ou$$

$$-Gly-Lys-Lys-Arg$$

ainsi que des peptides avec séquences partielles de la formule II, dans lesquelles jusqu'à 10 acides aminés de l'extrémité à terminaison en C font défaut ou de plus grands peptides, dans lesquels les peptides de la formule II représentent une séquence partielle, ainsi que des sels pharmaceutiquement acceptables de ces peptides pour la préparation de médicaments pour le traitement des dysfonctionnements érectiles chez les mammifères et chez l'homme.

2. Utilisation de CGRP humain de formule I

```
H-Ala-Cys-Asp-Thr-Ala-Thr-Cys-Val-Thr-His-Arg-

Leu-Ala-Gly-Leu-Leu-Ser-Arg-Ser-Gly-Gly-Val-Val-        (I)

Lys-Asn-Asn-Phe-Val-Pro-Thr-Asn-Val-Gly-Ser-Lys-

Ala-Phe-NH₂
```

pour la préparation de médicaments pour le traitement des dysfonctionnements érectiles chez les mammifères et chez l'homme, selon la revendication 1.

3.  Utilisation de peptides selon les revendications 1 ou 2, en combinaison avec une ou plusieurs substances actives choisies dans le groupe des substances actives formé de l'adénosine, des vitamines, des prostaglandines, des antagonistes du calcium, des bloqueurs de récepteurs $\alpha$, des agents de relaxation des muscles lisses, pour la préparation de médicaments pour le traitement des dysfonctionnements érectiles chez les mammifères et chez l'homme.

4.  Utilisation de peptides et de combinaisons de substances actives selon les revendications 1 à 3, caracérisée en ce que l'on utilise de 0,5 μg à 5 mg de peptides de selon les revendications 1 à 3 par dose unitaire pour le traitement des dysfonctionnements érectiles chez les mammifères et chez l'homme.